(19) Europäisches Patentamt
European Patent Office
Office européen des brevets

(11) Veröffentlichungsnummer : **0 259 396 B1**

# (12) EUROPÄISCHE PATENTSCHRIFT

(45) Veröffentlichungstag der Patentschrift : **24.07.91 Patentblatt 91/30**

(51) Int. Cl.$^5$ : **C07C 231/00,** C07C 233/90, C07C 271/06, C07K 1/00

(21) Anmeldenummer : **87901372.0**

(22) Anmeldetag : **27.02.87**

(86) Internationale Anmeldenummer : **PCT/DE87/00088**

(87) Internationale Veröffentlichungsnummer : **WO 87/05292 11.09.87 Gazette 87/20**

## (54) VERFAHREN ZUR HERSTELLUNG VON PEPTIDEN.

(30) Priorität : **07.03.86 DE 3608083**

(43) Veröffentlichungstag der Anmeldung : **16.03.88 Patentblatt 88/11**

(45) Bekanntmachung des Hinweises auf die Patenterteilung : **24.07.91 Patentblatt 91/30**

(84) Benannte Vertragsstaaten : **AT BE CH DE FR GB IT LI LU NL SE**

(56) Entgegenhaltungen : **The Journal of Organic Chemistry, vol. 37, no. 18, 8 September 1972 (American Chemical Society,USA), J.S. Paul Schwarz, "Preparation of acylic isoimides and their rearrangement rates to imides", pages 2906-2908, see page 2906**

(73) Patentinhaber : **BRADACZEK, Hans**
**Bergengruenstrasse 47**
**W-1000 Berlin 38 (DE)**
Patentinhaber : **GRUSZECKI, Wojciech**
**Berlepschstrasse 132**
**W-1000 Berlin 37 (DE)**
Patentinhaber : **GRUSZECKA, Maria**
**Berlepschstrasse 132**
**W-1000 Berlin 37 (DE)**

(72) Erfinder : **BRADACZEK, Hans**
**Bergengruenstrasse 47**
**W-1000 Berlin 38 (DE)**
Erfinder : **GRUSZECKI, Wojciech**
**Berlepschstrasse 132**
**W-1000 Berlin 37 (DE)**
Erfinder : **GRUSZECKA, Maria**
**Berlepschstrasse 132**
**W-1000 Berlin 37 (DE)**

EP 0 259 396 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft den in den Patentansprüchen gekennzeichneten Gegenstand.

Es ist bekannt, daß sich bestimmte, Peptidbausteine enthaltende Diacylamine zur Synthese von Peptiden eignen. So wurde beispielsweise das N-(N'-Phthalyl-glycyl)-N-diphenylacetyl-4-methylanilin zur Synthese von N-Phthalylglycyl-glycyl-glycin-ethylester verwendet (J. Am. Chem. Soc., 80, 1958, 4069). Demgegenüber haben die erfindungsgemäßen Diacylamine der formel IV den Vorzug, daß ihre Herstellung wesentlich weniger aufwendig ist. Zudem liefern sie bei der Umsetzung mit den Amino-Komponenten der allgemeinen formel V in überraschend hohen Ausbeuten und hoher Reinheit die Peptide der allgemeinen Formel I. Es ist für den Fachmann überraschend, daß man mithilfe der erfindungsgemäßen Diacylamine der allgemeinen Formel IV Peptide synthetisieren kann, denn die diesen Verbindungen entsprechenden in den Phenylgruppen unsubstituierten Verbindungen (d.h. von Verbindungen der allgemeinen Formel IV mit Y und Z jeweils in der Bedeutung von Wasserstoff) sind zur Peptidsynthese ungeeignet, wie literaturbekannt ist (Biochemistry 5, 1966, 2468 ff) und eigene Versuche zeigen.

Das erfindungsgemäße Verfahren wird in der Weise durchgeführt, daß man in einem ersten Reaktionsschritt eine Carbonsäure der allgemeinen Formel II mit einem Carbonsäureimidchlorid der allgemeinen Formel III umsetzt. Dieser Reaktionsschritt wird vorzugsweise in einem inerten Lösungsmittel in Gegenwart von Basen durchgeführt. Geeignete Lösungsmittel sind beispielweise niedere Alkohole, wie Methanol, Ethanol oder Isopropanol, niedere Carbonsäureester, wie Ethylacetat, polare Ether, wie Dioxan, Tetrahydrofuran, Glykolmonomethylether oder Glykolimethylether, Ketone wie Aceton, Methylethylketon oder Methylisobutylketon oder dipolare aprotische Lösungsmittel, wie Dimethylformamid, N-Methylacetamid, Sulfolan oder Hexamethylphosphorsäuretriamid. Geeignete Lösungsmittel sind ferner Gemische des obengenannten Lösungsmittel mit inerten unpolaren Lösungsmitteln, wie chlorierten Kohlenwasserstoffen (Dichlormethan, Trichlormethan oder Tetrachlorethan etc.) oder aromatische Kohlenwasserstoffe (Benzol, Toluol etc.). Geeignete Basen sind beispielsweise tertiäre Amine, wie Triethylamin, Tributylamin, N-Methylmorpholin oder N-Ethylpiperazin, Alkalimetalhydroxide wie Natronlauge oder Kalilauge, Alkalimetallcarbonate, wie Natriumcarbonat, Natriumhydrogencarbonat, Kaliumcarbonat oder Kaliumhydrogencarbonat oder Alkalimetallalkoholate, wie Natriummethylat oder Kaliummethylat. Dieser erste Reaktionsschritt wird üblicherweise bei einer Reaktionstemperatur von −20°C bis 100°C durchgeführt, wobei eine Temperatur von 0°C bis 30°C bevorzugt angewendet wird.

In der zweiten Stufe des erfindungsgemäßen Verfahrens werden die erhaltenen Diacylamine der allgemeinen Formel IV mit einem Amin der allgemeinen Formel V umgesetzt. Auch diese Stufe wird vorzugsweise in einem inerten Lösungsmittel durchgeführt. Als Lösungsmittel können die gleichen Solventien wie in dem ersten Verfahrensschritt verwendet werden. Die Reaktionstemperatur beträgt bei diesem Verfahrensschritt beispielsweise −20°C bis 100°C, wobei eine Temperatur von −10°C bis 30°C bevorzugt ist.

Das erfindungsgemäße Verfahren ist zur Synthese von Peptiden ebenso universell anwendbar, wie die vorbekannten Verfahren (Houben-Weyl "Methoden der organischen Chemie", Georg Thieme Verlag, Stuttgart BR-Deutschland, Band XV/1 "Synthese von Peptiden" Teil I, 1974 und Bans XV/2 "Synthese von Peptiden" Teil II, 1974). Ebenso wie bei den vorbekannten Verfahren ist es bei dem erfindungsgemäßen Verfahren zweckmäßig, die Aminogruppen, sowie gegebenenfalls auch vorhandene Hydroxygruppen, Thiogruppen und nicht umzusetzende Carboxylgruppen der als Ausgangsmaterialien verwendeten Carbonsäuren in an sich bekannter Weise zu schützen. Als Carbonsäuren, die sich zur Durchführung des erfindungsgemäßen Verfahrens eignen seien beispielsweise Peptid-Derivate der allgemeinen Formel IIa

$$V\text{-}(Am_1\text{-}Am_2...Am_x)\text{-}OH \qquad (IIa),$$

worin

$Am_1$, $Am_2$...$Am_x$ maximal 16 Reste einer natürlichen oder synthetischen Aminosäure darstellen und V eine Aminschutzgruppe, wie zum Beispiel die Benzyloxycarbonylgruppe oder die tert.-Butyloxycarbonylgruppe symbolisiert, genannt.

Als Reste $Am_1$, $Am_2$...$Am_x$ seien beispielsweise die in Hoppe-Seyler's Z. Physiol. Chem. 348, 1967, 256-261 aufgeführten Aminosäurn soweit deren gegebenenfalls vorhandene Hydroxygruppen, Thiolgruppen, Aminogruppen und nicht zur Umsetzung bestimmte Carboxylgruppen durch geeignete Schutzgruppen blockiert sind, genannt.

Bei den Aminen der allgemeinen Formel V werden die Carboxylgruppen und gegebenenfalls auch vorhandene Hydroxygruppen und Thiogruppen und nicht zur Umsetzung bestimmte Aminogruppen in an sich bekannter Weise geschützt.

Geeignete Amine sind beispielsweise Peptid-Derivate der allgemeinen formel Va

$$H(Am_1-Am_2...Am_x)-OW \qquad (V\ a),$$

worin

$Am_1$, $Am_2$...$AM_x$ die obengenannte Bedeutung besitzen und W eine Carboxylschutzgruppe, wie zum Beispiel niedere Alkoxygruppen mit maximal 4 Kohlenstoffatomen, wie die Methoxygruppe, Ethoxygruppe oder die tert.-Butyloxygruppe oder die Benzyloxygruppe oder ein Alkalimetallatom, wie Natrium oder Kalium beziehungsweise ein Trialkylammonium-Kation mit maximal 4 Kohlenstoffatomen pro Alkylgruppe symbolisiert.

Die zur Durchführung des erfindungsgemäßen Verfahrens benötigten Carbonsäureimidchloride der allgemeinen Formel III können aus den entsprechend substituierten N-Benzoylanilinen durch Umsetzung mit Chlorierungsmitteln wie Phosphorpentachlorid oder Thionylchlorid hergestellt werden.

Die nachfolgenden Ausführungsbeispiele dienen zur näheren Erläuterung des erfindungsgemäßen Verfahrens.

## I. Synthese von Carbonsfäureimidchloriden

Beispiel 1 :

12.0 g N-(2-Nitrobenzoyl)-anilin und 10.0 g Phosphorpentachlorid werden im Rundkolben mit Rückflußkühler gemischt und unter Ausschluß von Feuchtigkeit vorsichtig erwärmt. Beide Substanzen lösen sich unter starker HCl-Entwicklung auf. Die Lösing wird 1 Stunde zum Sieden erhitzt. Danach wird das Phosphoroxyclorid abdestilliert und das verbleibende braunfarbige Öl zweimal mit 10 ml wasserfreiem Toluol abgedampft. Der Rest von Toluol wird danach bei $10^{-2}$ Torr Druck und 80°C abgesaugt. Man erhält 12,8 g des N-Phenyl-N-(2-nitrobenzimid)-chlorid als brauns Masse.
(Literatur : R. A. Abramovitsch et. al., J. Org. Chem. 48, 4391 (1983)).

Beispiel 2 :

22.0 g N-(2-Chlorbenzoyl)-anilin wird mit 20.0 g Phosphorpentachlorid gemischt und wie im Beispiel 1 erhitzt. Nach der HCl-Entwicklung wird das Phosphoroxychlorid abdestilliert und das ölige Benzimidoylchlorid unter $10^{-2}$ Torr Druck durch Destillation gereinigt (Siedep. ca. 140°C). Das so erhaltene farblose Öl des N-Phenyl-N-(2-chlorbenzimid)-chlorid erstarrt in der Vorlage und schmilzt bei 54°C.
(Literatur : A. W. Chapman, J. Chem. Soc., 2296 (1926)).

Beispiel 3 :

23.0 g N-(2,6-Dichlorbenzoyl)-anilin (erhalten aus 2,6-Dichlorbenzoylchlorid und Anilin, Schmelzpunkt 176-177°) und 18.0 g Phosphorpentachlorid läßt man zusammen reagieren wie im Beispiel 2. Das ölige Produkt wird unter $10^{-2}$ Torr bei 135°C destilliert. Man erhält 21.0 g kristallines N-Phenyl-N-(2,6-dichlorbenzimid)-chlorid. Der Schmelzpunkt liegt bei 35°C.

Beispiel 4 :

15.0 g N-(2,6-Dichlorbenzoyl)-anilin wird in 20 ml Thionylchlorid 4 Stunden lang gekocht, danach der Überschuß an Chlorierungsmittel abdestilliert und das erhaltene ölige Produkt wird wie im Beispiel 3 weiter verarbeitet. Die Ausbeute beträgt 15.0 g N-Phenyl-N-(2,6-dichlorbenzimid)-chlorid.

## II. Darstellung von Diacylaminen

Beispiel 5 :

1 mM (= 175 mg) tert-Butyloxycarbonylglycin (BOC-GlyOH) wird in 2 ml Methanol gelöst und mit mM Triäthylamin (0,14 ml) neutralisiert. Die Lösung wird auf 0°C abgekühlt. Dazu tropft man 1, 1 mM (286,5 mg) N-Phenyl-N-2-nitrobenzimid)-chlorid (nach Beispiel 1) in 1 ml Toluol und rührt den Ansatz 1 Stunde bei Raumtemperatur. Die Lösungsmittel werden unter geringem Druck verdampft, der Rückstand in Essigester aufgenommen und nacheinander mit 0,5 n HCl, Wasser, gesättigter $NaHCO_3$-Lösung und wieder mit Wasser

gewaschen, über $MgSO_4$ getrocknet und im Vakuum zur Trockne eingedampft. Ausbeute : 409,0 mg N-(2-Nitrobenzoyl)-N-(N'-tert-butyloxycarbonyl-glycinyl)-anilin als gelber Schaum.

Beispiel 6 :

1 mM (189 mg) tert-Butyloxycarbonyl-L-alanin (BOC-L-Ala-OH) wird in 2 ml Essigester und 0,14 ml Triäthylamin gelöst. Nach Abkühlung im Eisbad fügt man 255 mg N-Phenyl-N-(2-chlorbenzimid)-chlorid hinzu und rührt die Lösung bei Raumtemperatur 30 Minuten. Danach wird die Lösung mit 0,5 n HCl, Wasser $NaHCO_3$-Lösung und nochmals mit Wasser gewaschen, über $MgSO_4$ getrocknet und unter geringem Druck verdampft. Man erhält 400 mg N-(2-Chlorbenzoyl)-N-(N'-tert-butyloxycarbonyl-L-alanyl)-anilin als weißen Schaum.

Beispiel 7 :

1 mM (= 265 mg) N-tert-Butyloxycarbonyl-L-phenylalanin (BOC-L-PheOH) wird in 5 ml Essigester gelöst und mit 1 n KOH neutralisiert. Bei +4°C wird es mit 1 mM (284 mg) N-Phenyl-N-(2,6-dichlorbenzimid)-chlorid versetzt und 4 Stunden bei Raumtemperatur gerührt, danach die wässrige Schicht abgetrennt. Die organische Phase wird zunächst mit $NaHCO_3$-Lösung und danach mit Wasser gewaschen, über $Na_2SO_4$ getrocknet und im Vakuum eingeengt. Die ölige Verbindung wird in Isopropanol kristallisiert. Ausbeute : 413 mg N-(2,6-Dichlorbenzoyl)-N-(N'-tert-butyloxycarbonyl-L-Phenylalanyl)-anilin vom Schmelzpunkt 154°C.

Beispiel 8 :

1 mM (= 175 mg) N-tert-Butyloxycarbonylglycin wird in 2 ml Aceton gelöst und mit 0,14 ml Triäthylamin neutralisiert. Die Lösung kühlt man im Eisbad. Danach werden 290 mg N-Phenyl-N-(2,6-dichlorbenzimid)-chlorid hinzugefügt und die Kühlung entfernt. Nach 3 Stunden wird das Aceton im Vakuum zur Trockne eingedampft und das neutrale Produkt wie im Beispiel 5 herausgezogen. Das Produkt wird danach in Toluol kristallisiert. Ausbeute : 410 mg N-(2,6-Dichlorbenzoyl)-N-(N'-tert-butyloxycarbonyl-L-phenylalanyl)-anilin vom Schmelzpunkt 109-111°C.

## III. Darstellung von Peptiden

Beispiel 9 :

Zu einer auf 4°C gekühlten Lösung von 1 mM (399 mg) N-(2-Nitrobenzoyl)-N-(BOC-Glycyl)-anilin (erhatene Substanz nach Beispiel 5) in 5 ml Aceton fügt man 1 mM (156 mg) Glycinäthylester-Hydrochlorid und 0,14 ml Triäthylamin hinzu. Danach wird die Lösung bei Raumtemperatur 1 Stunde gerührt. Man verdampft das Aceton bei geringem Druck. Die ölige Mischung wird mit 5 ml Benzoyl behandelt. Die ausgefallenen Kristalle werden abgesaugt und die Benzollösung zu dickem Öl eingeengt. Ausbeute : 270 mg ölige BOC-Gly-Gly-OEt ; die Substanz enthält noch 15-20 mg N-(2-nitrobenzoyl)-anilin.

Einen reinen Dipeptid-Ester kann man mit Hilfe der folgenden Prozedur darstellen :

a) Das rohe Produkt wird in Äther gelöst und von ungelösten Teilen durch Filtration getrennt, danach der Äther verdampft und die Substanz aus einer Mischung Benzol/Petrolether kristallisiert. So erhält man 215 mg Kristalle, die bei 60°C schmelzen.

b) Das Produkt wird durch Säulenchromatographie auf Silicagel gerinigt (Laufmittel : Benzol bzw. Benzol: Aceton 6 : 1). Ausbeute : 255 mg reiner BOC-Dipeptid-Ester.

c) Das rohe Produkt wird in 2 ml Aceton gelöst, mit 1 ml 1 n NaOH versetzt und 1 Stunde bei 20°C gerührt. Dann wird das Aceton verdampft und die wässrige Phase mit Essigester ausgewaschen, danach das Hydrolysat in Wasser mit Essigester bedeckt und mit 3 n Citrosäure (ph 3) angesäuret. Die organische Phase wird über $MgSO_4$ getrocknet und im Vakuum eingedampft. Ausbeute : 198 mg BOC-GLy-GLy-H. Der Schmelzpunkt liegt bei 125-127°C, Kristalle wurde aus Aceton kristallisiert.

d) Das rohe Produkt wird in 2 ml 1 n HCl in Essig gelöst und 45 min. bei 20°C belassen. Dann wird das Lösungsmittel im Vakuum abgezogen und der Rückstand aus Äthanol-Äther kristallisiert. Ausbeute : 178 mg H-Gly-Gly-OEt.HCl.

Beispiel 10 :

Zu einer auf 4°C gekühlten Lösung von 1 mM (175 mg) BOC-Gly-OH in 5 ml Essigester wird 0,14 ml Triäthylamin und 255 mg des unter Beispiel 2 beschriebenen N-Phenyl-N-(2-chlorbenzimid)-chlorid gegeben.

Nach 4 Stunden Rühren bei Raumtemperatur wird die Lösung wieder abgekühlt. Man gibt 1 mM (363 mg) H-Phe-Phe-OMe.HCl und danach 0,14 ml Triäthylamin hinzu. (Literatur : K. Eisele et al., Z. Phys. Chem. 356, 848 (1975)). Die Reaktionsmischung wird noch 4 Stunden bei Raumtemperatur gerührt, dann das ausgefallene Triäthylamin-Hydrochlorid mit wenig Wasser herausgelöst, die Essigesterphase über $NaSO_4$ getrocknet und im Vakuum eingeengt. Das BOC-Gly-Phe-Phe-OMe wird von 2-Chlorbenzanilid gemäß der in Beispiel 9a-9d beschriebenen Methoden gereinigt. Man erhielt beispielsweise gemäß der in Beispiel 9a oder 9b beschriebenen Methode 410-430 mg reines Produkt, das bei 153°-155°C schmilzt.

Beispiel 11 :

1 mM (165 mg) L-Phenylalanin wird in 1 n KOH in Methanol gelöst und auf −20°C abgekühlt. Danach fügt man 550 mg (1,3 mM) des unter Beispiel 8 beschriebenen aktiven N-BOC-Gly-N-(2,6-dichlorbenzoyl)-anilin hinzu und rührt 1 Stunde. Im Anschluß daran wird das Reaktionsgemisch 2 Stunden bei 0°C und eine weitere Stunde bei Raumtemperatur gerührt. Nach dieser Zeit wird das Methanol im Vakuum eingedampft, der Rückstand mit Essigester versetzt und mit Wasser herausgezogen. Die wässrige Phase wird mit 5 ml Essigester bedeckt und mit 3 n Citrosäure auf pH 3 angesäuert. Die Esterphase wird getrennt und über $MgSO_4$ getrocknet, dann im Vakuum zu weißem Schaum verdampft. Nach der Kristallisation in Aceton erhält man 202 mg BOC-Gly-1-Phe-OH, das sich bei 135°C zersetzt.

## Patentansprüche

1. Verfahren zur Herstellung von Peptiden der allgemeinen Formel I

$$R_1CO\text{-}NHR_2 \quad (I),$$

worin
$R_1CO$— die Carboxy-Komponente und
$R_2NH$ die Amino-Komponente eines Peptid-Bausteins bedeutet, dadurch gekennzeichnet, daß man eine Carbonsäure der allgemeinen Formel II

$$R_1COOH \quad (II),$$

worin
$R_1CO$— die obengenannte Bedeutung besitzt mit einem Carbonsäureimidchlorid der allgemeinen Formel III

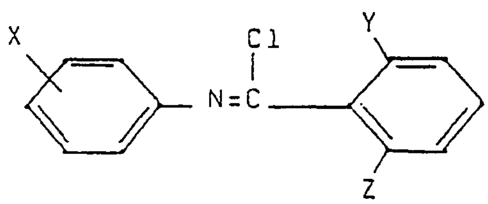 (III),

worin
X ein Wasserstoffatom, eine Alkylgruppe mit maximal 4 Kohlenstoffatomen, ein Halogenatom oder eine Nitrogruppe darstellt,
Y ein Halogenatom oder eine Nitrogruppe symbolisiert und
Z die gleiche Bedeutung wie Y besitzt oder ein Wasserstoffatom bedeutet umsetzt und das erhaltene Diacylamin der allgemeinen Formel IV

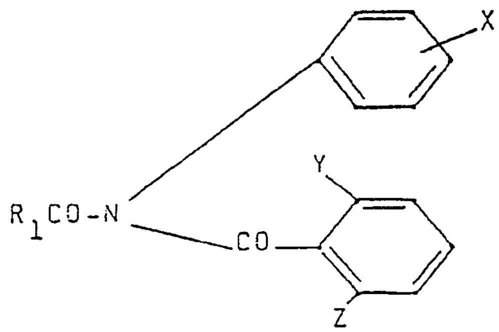

(IV),

worin
$R_1$, X, Y und Z die obengenannte Bedeutung besitzen mit einem Amin der allgemeinen Formel V

$R_2NH_2$ (V),

worin
$R_2NH$— die obengenannte Bedeutung besitzen, verknüpft.

2. Verfahren zur Herstellung von Peptiden gemäß Patentanspruch 1, dadurch gekennzeichnet, daß man als Carbonsäureimidchlorid eine Verbindung der allgemeinen Formel IIIa

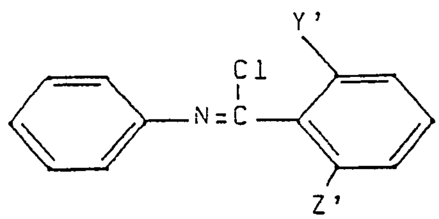

(III a),

worin
Y′ ein Chloratom oder eine Nitrogruppe und Z′ ein Wasserstoffatom oder Y′ und Z′ jeweils Chloratome oder Nitrogruppen bedeuten, verwendet.

3. Diacylamine der allgemeinen Formel IV

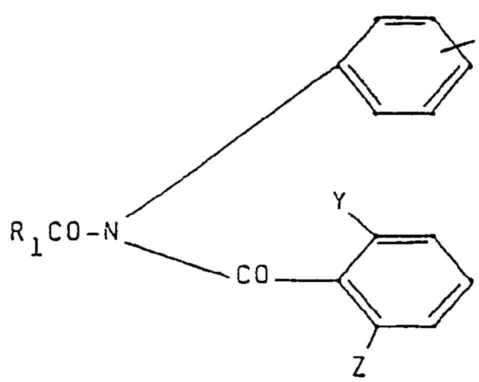

(IV),

worin
$R_1CO$—, X, Y und Z die im Patentanspruch 1 genannte Bedeutung besitzen.

4. N-(2-Nitrobenzoyl)-N-(N′-tert.-butyloxycarbonyl-glycyl)-anilin.

5. N-(2-Chlorbenzoyl)-N-(N′-tert.-butyloxycarbonyl-L-alanyl)-anilin.

6. N-(2,6-Dichlorbenzoyl)-N-(N′-tert.-butyloxycarbonyl-L-phenylalanyl)-anilin.

## Claims

1. Process for production of peptides of the general formula I

$$R_1CO\text{-}NHR_2 \quad (I),$$

in which
$R_1CO$— denotes the carboxy-component and
$R_2NH$ denotes the aminocomponent of a peptide fragment, which is characterized in that a carboxyl-acid of the general formula II

$$R_1COOH \quad (II),$$

in which
$R_1CO$ has the above mentioned meaning as reacted with a carboxylacidimidchloride of the general formula III

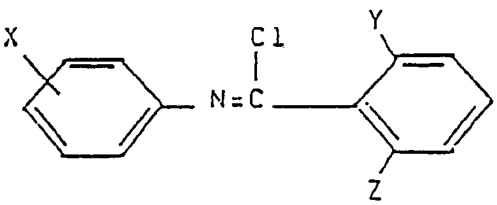

(III),

in which
X denotes a hydrogen atom, analcylgroup with maximal 4 C-atoms, a halogen atom or a nitrogroup,
Y denotes a halogen atom or a nitrogroup and
Z has the same meaning as Y or denote a hydrogen atom is reacted and the resulting diacylamine of the general formula IV

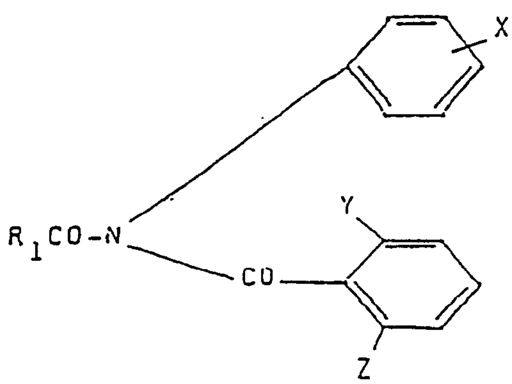

(IV),

in which
$R_1$, X, Y and Z denote the above mentioned meaning coupled with a amine of the general formula V

$$R_2NH_2 \quad (V),$$

in which
$R_2NH$ denotes the above mentioned meaning.

2. Process for the production of peptides according to claim 1, characterized in that as carboxylimidchloride is used as a compound of the general formula IIIa

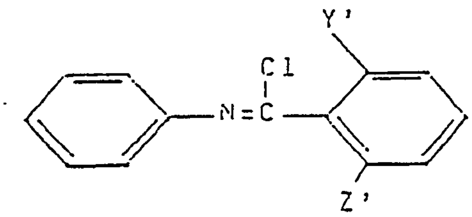

(III a),

in which
Y′ is a chloratom or a nitrogroup and Z′ a hydrogenatom or Y′ and Z′ are both chloratoms or nitrogroups.

3. Diacylamines of the general formula IV

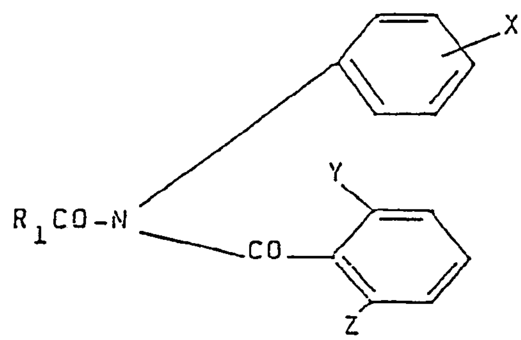

(IV),

in which
$R_1CO$—, X, Y and Z have the same meaning according to claim 1.

4. N-(2-nitrobenzoyl)-N-(N′-tert.-butyloxycarbonyl-glycyl)-aniline.

5. N-(2-chlorbenzoyl)-N-(N′tert.-butyloxycarbonyl-L-alanyl)-aniline.

6. N-(2,6-dichlorbenzoyl)N-(N′-tert.-butyloxycarbonyl-L-phenylalanyl)alanine.

**Revendications**

1. Procédé de fabrication de peptides selon la formule générale I

$$R_1CO\text{-}NHR_2 \quad (I),$$

dans laquelle
$R_1CO$— le composé Carboxy et
$R_2NH$ le composé amino d'un fragment peptide, procédé caractérisé de telle façon qu'on fait réagir un acide carboxylique de formule générale II

$$R_1COOH \quad (II),$$

dans laquelle
$R_1CO$— possède la signification indiquée ci-dessus avec un chlorure d'imide de formule générale III

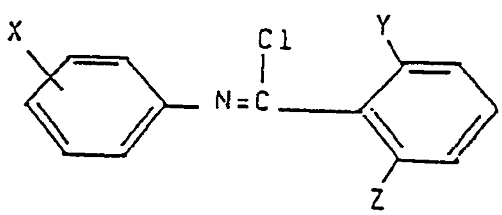

(III),

dans laquelle
X représente un atome d'hydrogène, un groupe acyle ayant 4 atomes de carbone au maximum, un atome halogène ou un groupe nitro

Y symbolise un atome halogène ou un groupe nitro et
Z a la même signification que Y ou symbolise un atome d'hydrogène pour obtenir une amine diacyle de formule générale IV

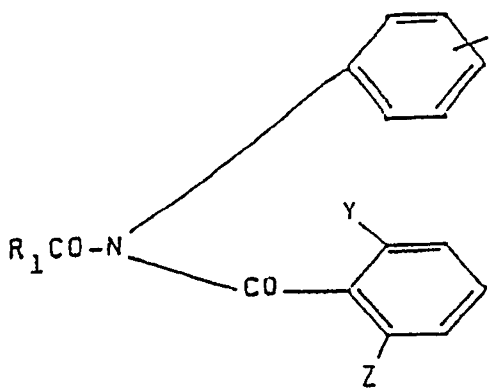

(IV).

dans laquelle
$R_1$, X, Y et Z possèdent les significations correspondantes indiquées ci-dessus et qu'on associe avec une amine de formule générale V

$$R_2NH_2 \quad (V),$$

dans laquelle
$R_2NH$— possède la signification indiquée ci-dessus.

2. Procédé de fabrication de peptides selon la demande de brevet 1, caractérisé de telle façon qu'on utillise comme chlorure d'imide un composé de formule générale IIIa

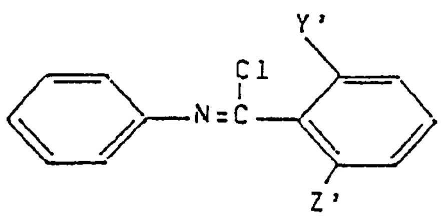

(IIIa).

dans laquelle
Y' représente un atome de chlore ou un groupe nitro et Z' un atome d'hydrogène ou Y' et Z' symbolisent soit des atomes de chlore ou des groupes nitro.

3. Amine diacyle de formule générale IV

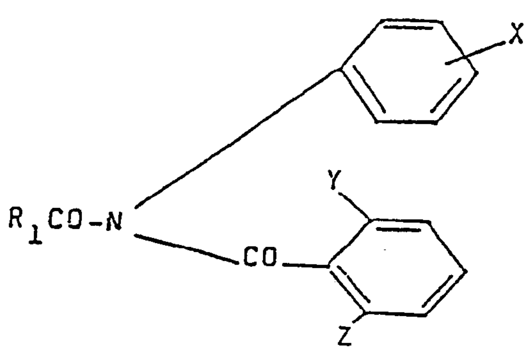

(IV).

dans laquelle
$R_1CO$—, X, Y et Z possèdent les significations données dans la demande de brevet 1.

4. N-(2-nitrobenzoyl)-N-(N'-tert.-butyloxycarbonyl-glycyl)-aniline.

5. N-(2-chlorbenzoyl)-N-(N'-tert.-butyloxycarbonyl-L-alanyl)-aniline.
6. N-(2,6-dichlorbenzoyl)-N-(N'-tert.-butyloxycarbonyl-L-phenylalanyl)-alanine.